# EUROPEAN PATENT APPLICATION

(11) **EP 4 302 677 A1**
(43) Date of publication of application: **10.01.2024**
(21) Application number: 22183911.1
(22) Date of filing: 08.07.2022
(51) Int. Cl.: A61B 1/005, A61B 8/12, A61B 8/00, A61B 1/267

(54) **A BENDING SECTION FOR AN ENDOSCOPE**

(71) Applicant: Ambu A/S, 2750 Ballerup (DK)
(72) Inventor: SØRENSEN, Morten, 2750 Ballerup (DK)
(74) Representative: AWA Denmark A/S

(57) **Abstract**

The endoscope comprises a proximal handle, an insertion cord extending from said handle towards a tip part (10) at the distal end of the endoscope. The insertion cord comprises a main tube (8), the tip part (10), and a bending section body (9). The bending section body (9) comprises a number of segments (13, 14, 15) interconnected by hinge members (16, 16'). The segments comprise a proximal end segment (13), a distal end segment (14) and a number of intermediate segments (15). The proximal end segment (13) and at least some of said number of intermediate segments (14) comprise pull-wire passages arranged in pairs in a plane perpendicular to said hinge plane. The pull-wires (11) in the passages are attached and secured to one of said intermediate segments (15).

## Description

The present disclosure relates to endoscopes, in particular but not exclusively to disposable EndoBronchial UltraSound endoscopes, normally abbreviated with the acronym EBUS endoscopes.

Like many other endoscopes, such EBUS endoscopes comprise a proximal handle from which an insertion cord extends. The insertion cord normally comprises a bendable main tube to the distal end of which a highly flexible bending section is connected. The bending section normally comprises a number of segments connected by hinge members so as to allow it to be articulated, that is to say bent out of a straight as-made configuration. In the straight as-made configuration the longitudinal axis of the bending section is normally aligned with the longitudinal axis of the main tube. The distal end of the bending section, in turn, is connected to a tip member with features providing desired functionalities such as illumination, vision, exit ports for tools, liquid instillation, fluid insufflation, suction etc. In EBUS endoscopes one of the features is an ultrasound transceiver allowing to look into the tissue behind e.g. the bronchial wall. Furthermore, in EBUS endoscopes, the tip member may comprise attachment means, such as recesses, for the attachment of a balloon surrounding the ultrasound transceiver. The balloon may then be filed with liquid from a port in the tip part so as to provide a good match of acoustic impedance to the surrounding tissue that is to be ultrasonically probed. The proximal handle is adapted to be gripped by a hand of an operator. The handle may comprise an operating member allowing control of the bending section, so that the insertion cord may be manoeuvred during insertion into a patient e.g. into the bronchia.

As e.g. known from US6409666 the ultrasound transceiver in the tip member emits and receives in a sideways direction i.e. at high angles transversely to the overall longitudinal direction of the endoscope, in the following understood as the centre axis of the main tube when in a straight as made configuration. That is to say, not bent by external forces. This allows the ultrasound transceiver to look through the bronchial wall and into the tissue behind, e.g. for locating lymph nodes. The vision camera on the other hand is more forward looking, allowing the operator to manoeuvre the tip of the endoscope to a desired position in the bronchia. The working channel at the exit port in the tip is also placed at an angle in order to allow a tool, in particular a biopsy needle, to exit at an angle allowing penetration of the bronchial wall into lymph nodes behind the bronchial wall for sampling while at the same time being visible by the ultrasound transceiver.

One problem in this respect is to press the emission face of the ultrasound transceiver into proper engagement with the bronchial wall, *inter alia* to avoid air filled gaps between the emission face and the bronchial wall that will cause sound reflections because of poor match of acoustic impedance between the various media, e.g. air vs. waterfilled tissue. Although this may partially be remedied by using a liquid fillable balloon around the tip a good engagement force is still necessary.

Another problem is that the surface of the ultrasound transceiver is curved and different parts along the length of the emission face will engage the tissue depending on the dimensions of the surrounding bronchial passage.

Furthermore, since the bronchia branches into narrower and narrower passages, it may often be difficult for more proximal parts of the bending section or the main tube to properly engage a bronchial wall. Without a more proximal part also abutting the wall it is not possible to provide any proper engaging force with the tip. This may also influence the force and which place on the surface engages the ultrasound transducer.

As known from EP2572643 and US933961, one way to overcome this has to been to provide the part of the tip that accommodates the ultrasound transducer with a predetermined angle, i.e. that the back of the tip opposite the front face of the curved ultrasound transceiver has an angle with respect to the centre axis. This, however, does not solve all of the above problems satisfactorily. *Inter alia* the crosswise dimensions of the distal end of the endoscope increase because of the tip part being bent out of the plane.

Based on this it is the object of the present disclosure to provide an endoscope having a bending section that overcomes these drawbacks.

According to a first aspect of the present disclosure this object is achieved by an endoscope comprising a proximal handle, an insertion cord extending from said handle towards a distal end of the endoscope, where said insertion cord comprises a main tube, a tip part, and a bending section body arranged between said main tube and said tip part, where the bending section body comprises a number of segments interconnected by hinge members arranged in a hinge plane, said segments comprising a proximal end segment, a distal end segment and a number of intermediate segments, where the proximal end segment and at least some of said number of intermediate segments comprise pull-wire passages, where wherein said pull-wires are attached and secured to one of said intermediate segments.

By attaching the pull-wires to an intermediate segment only, rather than to the distal end segment, the hinge towards the distal end segment may bend independently of the bending of the remainder of the bending section. Accordingly, when the bending section bends the intermediate segments in a direction towards a bronchial wall, the wall may press the distal end segment to which the tip part is attached into the opposite direction, thus ensuring a better, i.e. more parallel alignment between the wall and the tip part. That is to say, better control the emission and reception direction of the ultrasound transceiver with respect to a specific direction towards a target behind the wall, if the tip part comprises an ultrasound transceiver.

According to a second aspect of the disclosure the object is achieved by a system comprising a display unit and an endoscope according to any one of the preceding claims connectable to said display unit.

By using the endoscope according to the first aspect in such a system, better visual and/or ultrasonic targeting of objects of interest behind a bronchial wall may be achieved.

According to an embodiment of the first aspect of the disclosure, the pull sire passages arranged in pairs in a plane perpendicular to said hinge plane. This allows efficient conversion of the pull into torque about the hinges.

According to an embodiment of the first aspect of the disclosure, said number of intermediate segments comprise a first set of intermediate segments through which the pull-wires pass, and a second set of intermediate segments through which the pull-wires do not pass, said second set of intermediate segments being arranged between said first set of intermediate segments and said distal end segments, wherein the pull-wires are connected to the most distal intermediate segment of the first set of segments. This allows even better alignment of the tip part.

According to another embodiment of the first aspect of the disclosure, the number of intermediate segments in said second set of intermediate segments is selected from the group comprising 1, 2, 3, 4 or 5. This has been found to be sufficient when the tip part comprises said ultrasound transceiver.

According to an embodiment of the first aspect of the disclosure, the bending section body is provided as an integrally moulded item and the hinges are provided as part of the integrally moulded item. This takes advantage of the fact that such integrally moulded hinges are elastic with a certain resiliency allowing them to transfer a force from the actively controlled bending section segments to the passive distal end segment and any passive intermediate segments. This in turn ensures good engagement between the tip part and the bronchial wall.

According to an embodiment of the first aspect of the disclosure, the hinge between the distal end segment is thicker than at least some of the hinges between intermediate segments. This allow the hinge to be more resistant to bending and hence increases the force with which the tip with the ultrasound transceiver may be pressed be pressed against the bronchial wall.

According to an embodiment of the first aspect of the disclosure, the tip part comprises an ultrasound transceiver. Though the alignment and engagement with the bronchial wall may have other uses, it is especially advantageous to ensure good contact with no or few acoustic reflections between the ultrasound transducer and the tissue of and behind the bronchial wall. This in turn yields good ultrasound images.

According to an embodiment of the second aspect of the disclosure, the display unit comprises an integrated display. Having an integrated display gives lesser items to handle in the set-up for patient examination.

According to an embodiment of the second aspect of the disclosure, the system further comprises an ultrasound control box for sending signals to an ultrasound transceiver and receiving signals from an ultrasound transceiver. This provides interchangeability of not only the ultrasound control box but also of the display device and other parts of the system, and they may not depend on each other.

According to an embodiment of the second aspect of the disclosure, the ultrasound control box comprises image processing electronics. This allows the ultrasound box to be a self-contained unit with or without integrated display.

The disclosure will now be made in greater detail based on non-limiting exemplary embodiments and the drawings, on which:
Fig. 1 shows a system according to the disclosure comprising an EBUS endoscope, a display unit and an ultrasound box,
Fig. 2 shows an exploded side view of the main tube, bending section and a tip part of the EBUS endoscope of Fig. 1,
Fig 3 shows an isometric view from the distal end of a bending section body of the bending section of Fig. 2,
Fig. 4a shows the bending section body of Fig. 3 in isometric view but from a slightly different angle and in partial section,
Fig. 4b shows the two pull-wires for operating the bending section of Fig. 4a,
Fig. 5 shows an enlarged side view of the tip part of the tip part of Fig. 2,
Fig. 6 shows a cross-section of the bending section of Fig. 2, and
Fig. 7 schematically shows how the segments of the bending section would bend within a duct or passage, such as in the bronchia.

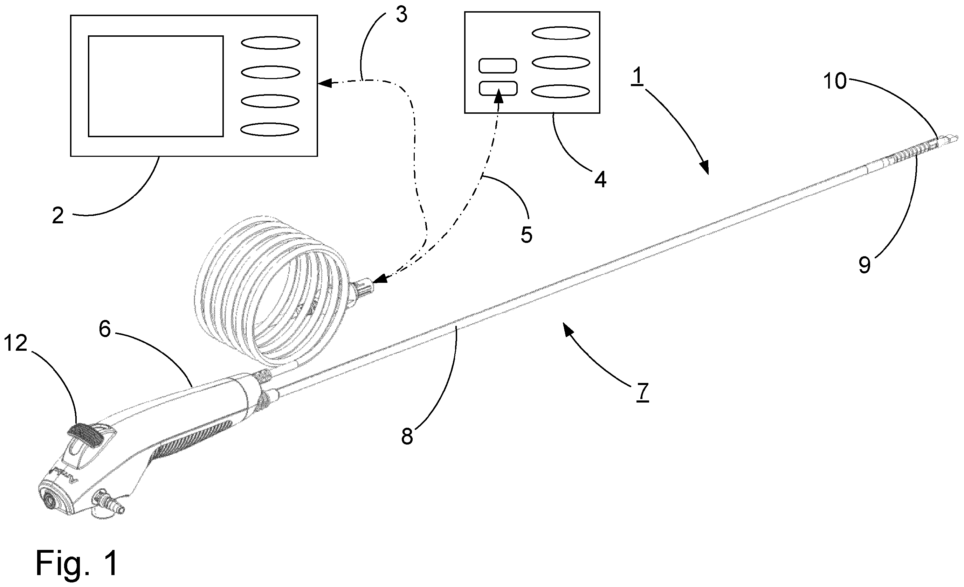

Turning first to Fig. 1 an EBUS endoscope 1 according to the disclosure is shown. The endoscope 1, as shown, forms part of a system further comprising a display device 2 to which the endoscope 1 is connectable as indicated with the dashed double arrow 3. Evidently, the connection to the display device 2 need not be direct but may be via a display unit with image processing capabilities to which the display 2 is connected. When, as envisaged, the endoscope is an EBUS endoscope the system further comprises an ultrasound control box 4 to which the endoscope is also connectable, as indicated by the double arrow 5. The ultrasound control box 4 comprises signal processors and other electronics for sending signals to the ultrasound transceiver and receiving signals therefrom. The electronics may also include processors as CPUs or FPGAs for processing the ultrasound images to be displayed. The ultrasound image could be sent to a separate display, or be sent to the image display, e.g. as picture-in-picture, if the display device has only a single screen, or the ultrasound control box may have its own integrated display.

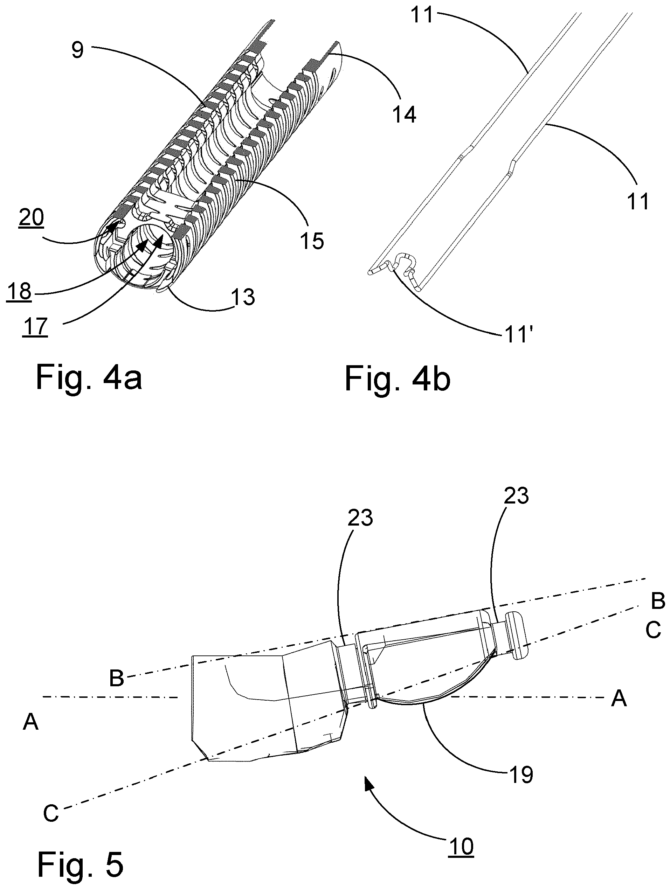

The endoscope 1 comprises a proximal handle 6 from which an insertion cord 7 extends towards to the distal end of the endoscope 1. The insertion cord 7 comprises several parts, such as a main tube 8, a bending section comprising a bending section body 9, and a tip part 10. Pull-wires 11 for actively bending the bending section body 9, are attached to the bending section body 9 and run from the bending section body 9 through the main tube 8 to an operating member 12 where they are also secured, thus allowing the bending section body 9 to be bent in different directions by tensioning or slacking the pull-wires 11 in a *per se* well known manner. This not only allows the distal end of the endoscope to be manoeuvred to a target site but also to force the ultrasound transceiver 21, seen in Fig. 5, of the tip part into engagement with a tissue, such as a bronchial wall, at the target site. The bending section body 9 is an open construction and is therefore normally covered by a sheath which, however, has been entirely omitted in the figures so as not to disturb the illustration of the bending section.

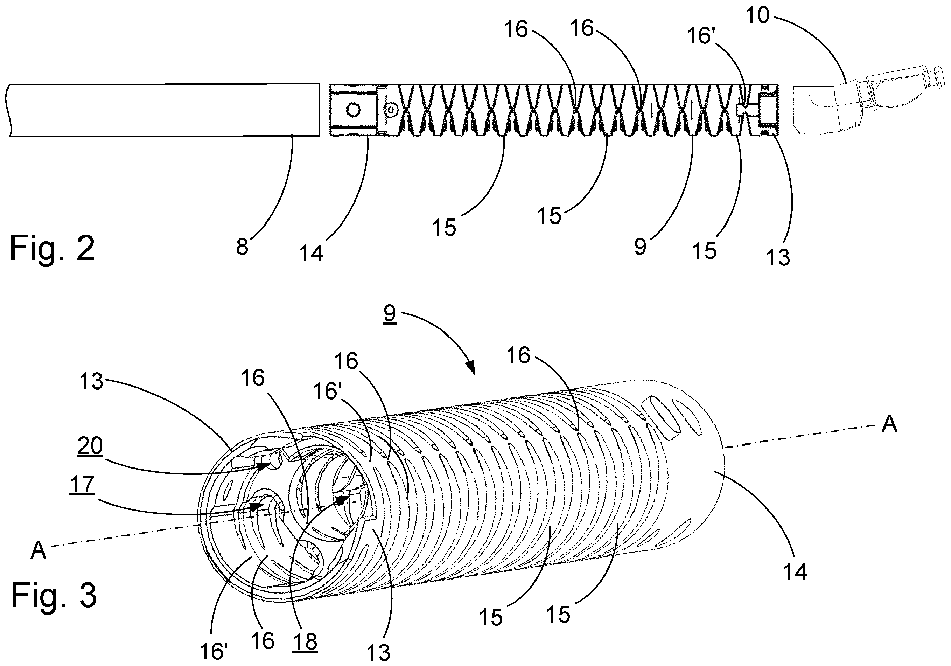

In Fig. 2 the tip part 10, the bending section body 9 and a distal part of the main tube 8 are shown in exploded view i.e. before the tip part 10 is adhered to the distal end segment 13 of the bending section body 9 and the main tube is adhered to the proximal end segment 14 of the bending section 9. As can be seen, the proximal end segment 14 of the bending section body 9 and the distal end segment 13 of the bending section body 9 are separated by a number of intermediate segments 15. The segments 13, 14, 15 are interconnected by short bridges of material serving as hinges 16, 16'. The bending section is preferably integrally moulded as a single piece member of one and the same polymer material. The elastic properties of the polymer material allow the short bridges to exhibit some resiliency so that the hinges 16, 16' bias the bending section towards a straight configuration as shown in Figs. 1, 2, 3, 4a and 6.

As can better be seen from Fig. 3 the intermediate segments comprise two larger passages 17, 18 arranged symmetrically around the hinge plane so that the hinge between intermediate segments comprise three bridges, two circumferential and one coincident with the extension of the wall between the passages in the intermediate hinges. One passage 18 is preferably circular in order to accommodate a working channel tube (not shown) whereas the other passage 17 is preferably somewhat kidney shaped and suitable for accommodating the quite substantial number of electrical wires for the array of transducers in the ultrasound transceiver 21, as well as optical fibres or electrical wires for illumination and electronic imaging of the visual part of the endoscope 1.

The intermediate segments 13 furthermore comprises a pair of smaller passages 20 (only one visible in Fig 3) for the pull-wires 11 used to bend the bending section 9. The smaller passages 20 and the pull-wires 11 are arranged in a plane crosswise to the plane in which he hinges 16 lie.

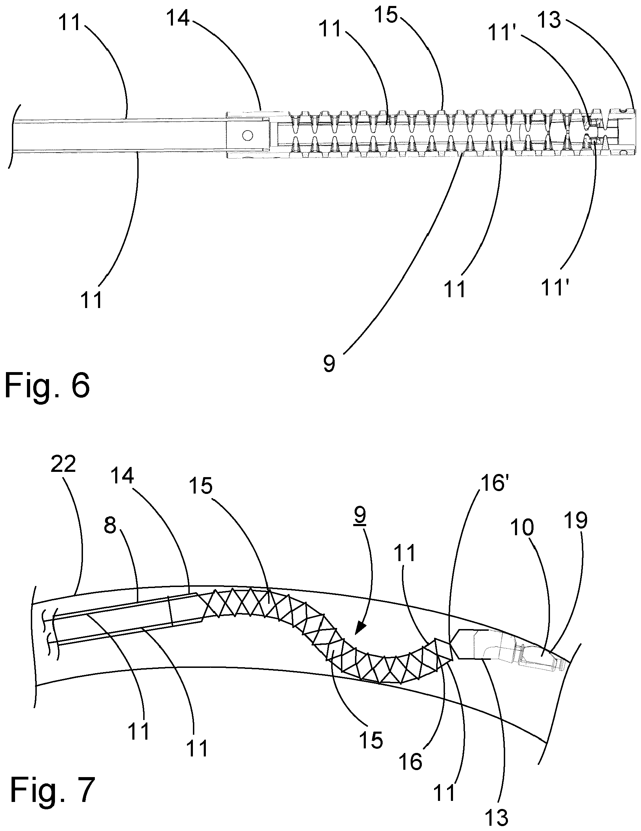

As can be seen from Figs. 4a and 4b, the pull-wires 11 are in the illustrated embodiment attached to the most distal intermediate segment 16. In practice the pull-wires 11 are as shown constituted by a single wire threaded through both passages 20 in the most distal intermediate segment 15 but since they are attached to the intermediate segment 15 it cannot be distinguished by form pulling at the proximal ends whether there is two individually secured wires or two free ends of a single wire. So, for practical and kinematic purposes there are two pull-wires 11. As can be seen from Fig. 4b the interconnection 11' of the two pull-wires 11 is wrapped though a series of bends to secure firm attachment to the intermediate segment 15. The interconnection 11' and the pull-wires 11 are preferably further secured using an adhesive. This can also be seen in the cross-sectional view of Fig. 6.

As explained, the pull-wires 11 are not attached to the distal end segment 13. Pulling the pull-wires 11 will thus not force any bending of the hinge 16'. The hinge 16' will only bend if an external force in a crosswise direction of the hinge plane is applied to the distal end segment 13. This will happen if the hinge segment 13 itself or the tip part 10 which is rigidly attached to the end segment 13, is forced into engagement with an external object, such as a bronchial wall 22, as can be seen schematically in Fig 7.

Fig. 4a shows an exploded view in partial cross-section of the bending section body 9 of Fig. 3. The cross-section is taken along the passage 17 suitable for accommodating the quite substantial number of electrical wires for the array of transducers in the ultrasound transceiver 21, as well as optical fibres or electrical wires for illumination and electronic imaging of the visual part of the endoscope 1. As can be seen this passage 17 does not run as a separate passage along all the intermediate segments, but rather joins the passage 18 for the working channel tube 18 form a single passage accommodating both. This joining of the passages would also be possible in the previously described embodiment of Fig 3.

To yield a good abutment force of the ultrasound transceiver against the bronchial wall 22 the hinge 16' connecting the distal end segment 13 to the intermediate segments is preferably more resilient than the hinges 16 between the intermediate segments 15. This may be achieved by the hinge 16' being made wider in the circumferential direction of the bending section body 9 than the circumferential width of the intermediate segments 15, as can be seen in Figs. 2 and 3.

In this respect it should be noted that the pull-wires 11 do not need to be attached to the most distal intermediate segment 15 as shown in Figs. 4 and 6. Rather it could be attached to a more proximal intermediate segment 15 so that not only the distal end segment but also one or more of the intermediate segments 15 will be able to bend under external force rather than under the direct influence of the pull-wires 11. In other words, the bending section would comprise a set of segments 14, 15 forming an active bending section part and a set of segments 13, 15 forming a passive bending section part. Accordingly, the intermediate segments 15 would comprise a proximal set of intermediate segments 15 forming part of the passive active bending section, and a distal set of intermediate segments 15 forming part of the passive bending section. Depending on the specific application for the endoscope 1, the passive bending section part could then include just the most distal intermediate segment 15 or up to e.g. five intermediate segments 15 towards the distal end segment 13. The hinges 16 between these intermediate segments 15 could also have increased resiliency, e.g. by making them the same width at the hinge 16' between the most distal intermediate segment 15 and the distal end segment 13.

Fig. 5 shows a more detailed sideview of the tip part 10. During assembly of the endoscope 1, the tip part 10 is adhered to the distal end segment 13 of the bending section body 9 so that the axis A-A shown in Fig 5 is aligned with the axis A-A shown in Fig. 3. The axis A-A also constitutes the centre axis of the generally cylindrical main tube 8 and defines the overall longitudinal axis of the endoscope 1. As can be seen the tip part 10 is asymmetrical in the sense that the curved emission and reception surface of the ultrasound transceiver 21 lies more or less on is at least intersected by the central axis A-A. This provides ultrasound transceiver with an emission or reception field pointing not only sideways but also somewhat in the forward direction, i.e. with respect to the insertion direction of the endoscope 1, beyond the distal end. This is illustrated by the chord C-C between the ends of the emission and reception surface of the ultrasound transceiver 21, as well as the inclination of the opposite side of the tip part 10, indicated by the axis B-B, with respect to the centre axis A-A.

As can be seen the tip part 10 comprises a pair of recesses 23 adapted to receive ends of a generally tubular balloon (not shown) which may be filled with a suitable liquid, such as water or aqueous saline solution to avoid any air trapped between the ultrasound transceiver 21 that could provide undesired sound reflections due to poor match of acoustic impedance between the ultrasound transceiver 21 and tissue, such as a bronchial wall 22. In other embodiments, there may be only a single recess 23 or groove receiving the balloon.

Fig. 7 is a highly schematic illustration of the engagement of the ultrasound transceiver 21 in the tip part 10 with the bronchial wall 22. It is emphasized that the illustration is schematic and inter alia does not show any deformation of the bronchial wall 22 from the pressure applied thereto by the bending section body 9 and the tip part 10. As can be seen the hinge 16' between the distal end segment 13 and the last, most distal intermediate segment 15 bends in the opposite direction of the hinge 16 between most distal segment 15 and the penultimate segment 15 before it.

This allows the tip part 10 to obtain a good engagement position with respect to the bronchial wall 22 and with respect to the procedure to be performed, *inter alia* involving the capture of ultrasound and optical images, as well as the taking of biopsies or the like via the working channel. The rigidity of the hinges 16 arising from the fact that they rely on bending of the material form which the bending section body 9 is made, rather than low friction pivotal hinges as often used in reusable endoscopes, thus helps securing this good engagement.

Although the above description has been given based on an EBUS endoscope, the skilled person will understand that the concept of one or more passive links in the bending section of an endoscope is not limited to EBUS endoscopes but will be applicable for any endoscope where there may be an interest in a firm aligned sideways abutment of the tip against an object, such as a cavity wall. Such endoscopes may be with or without ultrasound capabilities and could *inter alia* include EUS endoscopes for gastric purposes or duodenoscopes.

## Claims

1. An endoscope comprising a proximal handle, an insertion cord extending from said handle towards a distal end of the endoscope, where said insertion cord comprises a main tube, a tip part, and a bending section body arranged between said main tube and said tip part, where
the bending section body comprises a number of segments interconnected by hinge members arranged in a hinge plane, said segments comprising a proximal end segment, a distal end segment and a number of intermediate segments, where
the proximal end segment and at least some of said number of intermediate segments comprise pull-wire passages,
wherein said pull-wires are attached and secured to one of said intermediate segments.

2. An endoscope according to claim 1, wherein the pull-wire passages arranged in pairs in a plane perpendicular to said hinge plane.

3. An endoscope according to any one of the preceding claims, where said number of intermediate segments comprise a first set of intermediate segments through which the pull-wires pass, and a second set of intermediate segments through which the pull-wires do not pass, said second set of intermediate segments being arranged between said first set of intermediate segments and said distal end segments, wherein the pull-wires are connected to the most distal intermediate segment of the first set of segments.

4. An endoscope according to claim 3, wherein the number of intermediate segments in said second set of intermediate segments is selected from the group comprising 1, 2, 3, 4 or 5.

5. An endoscope according to any one of the preceding claims, wherein the bending section body is provided as an integrally moulded item and the hinges are provided as part of the integrally moulded item.

6. An endoscope according to any one of the preceding claims, wherein the hinge between the distal end segment is thicker than at least some of the hinges between intermediate segments.

7. An endoscope according to any one of the preceding claims wherein the tip part comprises an ultrasound transceiver.

8. A system comprising a display unit and an endoscope according to any one of the preceding claims connectable to said display unit.

9. A system according to claim 8, wherein the display unit comprises an integrated display.

10. A system according to any one of claims 8 to 9, further comprising an ultrasound control box for sending signals to an ultrasound transceiver and receiving signals from an ultrasound transceiver.

11. A system according to claim 10, wherein the ultrasound control box comprises image processing electronics.
